# EUROPEAN PATENT APPLICATION

(11) **EP 4 556 568 A1**
(43) Date of publication of application: **21.05.2025**
(21) Application number: 23210482.8
(22) Date of filing: 17.11.2023
(51) Int. Cl.: C12P 13/04, C12N 9/88

(54) **BIOCATALYTICAL METHODS FOR PRODUCING ETHYLENEDIAMINE-N,N'-DISUCCINIC ACID**

(71) Applicant: BASF SE, 67056 Ludwigshafen am Rhein (DE)
(72) Inventor: Fleck, Christian, 67056 Ludwigshafen am Rhein (DE); Bohnenkamp, Anna Christina, 67056 Ludwigshafen am Rhein (DE); Muenkel, Ronja, 67056 Ludwigshafen am Rhein (DE); Maurer, David Martin, 67056 Ludwigshafen am Rhein (DE); Mahler, Eva, 67056 Ludwigshafen am Rhein (DE); Reinoso Garcia, Marta, 67056 Ludwigshafen am Rhein (DE); Willrodt, Christian, 67056 Ludwigshafen am Rhein (DE); Hoschek, Anna, 67056 Ludwigshafen am Rhein (DE)
(74) Representative: Eisenführ Speiser

(57) **Abstract**

The present invention lies in the field of biocatalysis and provides methods for producing ethylenediamine-*N*,*N*'-disuccinic acid as well as biocatalyst mixtures for use in the production of ethylenediamine-*N,N*'-disuccinic acid.

## Description

The present invention lies in the field of biocatalysis and provides methods for producing ethylenediamine-*N,N*'-disuccinic acid as well as to biocatalyst mixtures for use in the production of ethylenediamine-*N,N*'-disuccinic acid.

Enzymes are protein-based biocatalysts widely used in a variety of different industries. They can be specific and enantioselective catalysts and can be employed under milder reaction conditions in comparison to traditional catalysts. They, for example, can be catalytically active at lower temperatures, which can result in an overall reduction of energy consumption of production processes. Additionally, enzymes are biodegradable and therefore increasingly used in various applications as sustainable alternative to petrochemistry.

Ethylenediamine-N,N'-disuccinic acid (EDDS) is a chelating agent that can bind metal ions in a stable chelator complex. EDDS is biodegradable, has a low toxicity and is therefore an attractive alternative to traditional chelating agents such as ethylenediaminetetraacetic acid (EDTA). EDTA is a synthetic chelator, currently widely used in industry, with very similar structure and properties to EDDS. However, EDTA is not biodegradable. For achieving a more sustainable industry, it is desired to use chelators, which are biodegradable.

EDDS is produced by reacting e.g. maleic acid or fumaric acid with ethylenediamine as described in US3158635A. Furthermore, it is also possible to produce EDDS by reacting fumaric acid and ethylenediamine under the aid of bacteria expressing an EDDS synthase *(*Takahashi et al., "Production of (S,S)-Ethylenediamine-N,N'-disuccinic Acid from Ethylenediamine and Fumaric Acid by Bacteria", Bioscience, Biotechnology, and Biochemistry, 63:7, 1269-1273, 1999)*.*

The patent JP4253195B2 discloses a biocatalyst for producing EDDS and methods for using the disclosed enzyme to produce EDDS. As the needed amount of EDDS is steadily increasing, it is desired to improve the yield of EDDS synthesis. One way of achieving this goal is to decrease the activity of fumarase, which hydrates the substrate fumaric acid and thus deprive the amount of substrate available for the formation of EDDS.

The patent EP0927762B1 discloses a method for inactivation of fumarase activity in a microorganism expressing an EDDS lyase to improve the yield of EDDS by decreasing the formation of by-products. An alkaline solution in the presence of at least one salt with a concentration of 5 mM to 1000 mM is used.

The Japanese patent application JP2020162539A discloses a method for heat inactivation of microorganisms expressing an ethylenediamine lyase for producing EDDS while the enzymatic activity is maintained. The heat inactivation is done by keeping the microorganisms at 54°C to 60°C. No EDDS yield improvement of the enzyme is disclosed.

As the need for biodegradable chelators like EDDS rises continuously, methods are needed, which provide a high yield.

Thus, the primary object of the present invention was the provision of biocatalytic methods for the production of EDDS, which give a high yield and overall efficient synthesis process.

This primary object was solved in terms of the present invention by providing a method for producing ethylenediamine-N,N'-disuccinic acid (EDDS) comprising or consisting of the steps:
i. cultivating at least one cell expressing an EDDS synthase,
ii. obtaining a cell culture broth and optionally separating the cell from the culture medium to obtain a whole-cell biocatalyst,
iii. subjecting the obtained cell culture broth or the obtained whole-cell biocatalyst to a cell disruption step,
iv. obtaining a biocatalyst mixture,
v. providing at least one substrate selected from the group consisting of fumaric acid, fumarate, maleic acid, malic acid and maleic anhydrate, or a mixture thereof and ethylenediamine,
vi. mixing the obtained biocatalyst mixture of step iv. with the at least one substrate provided in step v.,
vii. incubating the mixture of step vi. at conditions suitable for the formation of EDDS,
viii. obtaining a mixture containing EDDS,
ix. optionally purifying the obtained mixture of step viii.

An "EDDS synthase" in terms of the present invention is an enzyme, which catalyzes the formation of ethylenediamine-N,N'-disuccinic acid and has a broad substrate spectrum. Other terms of this enzyme are known to the person skilled in the art and include e.g. the term EDDS lyase.

Step i. of the method according to the present invention includes culturing the at least one cell at conditions suitable for growth and expression of an EDDS synthases. Such suitable culture conditions are known to the person skilled in the art and need to be adapted to the at least one cell to be cultured.

The at least one cell preferably does not naturally express the EDDS synthase. Thus, the host cell is a recombinant host cell and includes a nucleic acid molecule encoding an EDDS synthase as described herein.

A "cell culture broth" in terms of the present invention describes preferably a mixture of cultivated cells and cell culture medium, wherein the cells are grown in the cell culture medium in step i. of the method according to the invention. The optional step of separating the cell from the cell culture medium preferably includes at least one separation technique selected from the group consisting of filtration, centrifugation, sedimentation and chromatography. The step of separating also preferably includes a partial or total separation of the cells and the cell culture medium and the concentration of the cell culture broth by enhancing the concentration of cells and partially removing the cell culture medium. Furthermore, the term "cell culture broth" also preferably includes mixtures of separated cells and freshly added cell culture medium, which was not used in step i. for cultivating the cells. Also, mixtures are included, which comprises cells and a suitable buffer or water for storing the cells expressing the biocatalyst. Preferably, stabilizers may be added for storing the cells expressing the biocatalyst, preferably selected from the group consisting of sugars, preferably cellulose or trehalose, polyols, preferably glycerol and sugar alcohols, preferably sorbitol.

A "cell disruption step" in terms of the present invention includes all methods, which are able to (partially) disrupt or permeabilize the cell membrane and/or the cell wall (if the cultured cell comprises a cell wall) and inactivate cell metabolism. This inactivation includes complete inactivation and partial inactivation.

It was surprisingly found in terms of the present invention that a cell disruption step, which leads to a full or partial disruption of the cell, was suitable to enhance the activity of the expressed EDDS synthase in the subsequent synthesis of EDDS. Whenever the present invention refers to the activity of the EDDS synthase, the formation of EDDS over time is meant based on the substrate volume and/or the used amount of biocatalyst. The activity of the EDDS synthase is equivalent to the performance of the EDDS synthase. This was especially surprising as it was found that the cell disruption step is suitable to reduce the intrinsic activity of the biocatalyst for fumaric acid degradation, which is normally expressed by the at least one cell as part of the cell's citrate cycle and thus improves the substrate availability for the EDDS synthase. Furthermore, the cell disruption step has the effect that the at least one cell is (partially) inactivated.

The biocatalyst mixture obtained in step iv. comprises the disrupted cells and cell culture medium and optionally further components. Preferably, the biocatalyst mixture comprises the partially disrupted cells and cell culture medium and optionally further components. Also preferably, the biocatalyst mixture comprises disrupted, partially disrupted and/or permeabilized cells and cell culture medium and optionally further components. Instead of cell culture medium, a biocatalyst mixture preferably comprises water, a buffer and optionally further components. Preferably, the obtained biocatalyst mixture can be further processed by suitable downstream operations such as concentration, dilution, dialysis, or crystallization.

The at least one substrate provided in step v. is selected from the group consisting of fumaric acid, fumarate, maleic acid, malic acid and maleic anhydrate, or a mixture thereof and ethylenediamine.

Preferably, also provided are bi- or trivalent cations selected from the group consisting of alkaline earth metals, iron, zinc, copper, nickel, aluminium, titanium and manganese. The provided cations form a complex with the produced EDDS and thus shift the reaction equilibrium to the product.

Chelating agents such as EDDS and their respective alkali metal salts are useful sequestrants for metal ions (e. g. metal ions such as Mg2+, Ca2+, Ba2+, Cu2+, Fe2+, Zn2+, Fe3+). Preferably, the EDDS synthesized using the EDDS synthase of the present invention, is in the form of a metal-EDDS complex, preferably in the form of an Mg-EDDS complex.

EDDS possesses two chiral centers, leading to the general possibility of generating three stereoisomeric forms, namely (R,R)-EDDS, (S,S)-EDDS and achiral meso-(R,S)-EDDS. Preferably, the EDDS synthase of the present invention produces predominantly (S,S)-EDDS. In a preferred embodiment, the EDDS synthase of the present invention produces (S,S)-EDDS with optical purity exceeding at least 90%, at least 91%, at least 92%, at least 93%, at least 94%, at least 95%, at least 96%, at least 97%, at least 98%, at least 99% enantiomeric excess (ee) or 100% (S,S)-EDDS.

The mixing in step vi. is conducted by contacting the provided substrate and optionally the provided ions with the enzymes by e.g. providing them as part of one reaction mixture and/or contacting them via the use of a membrane and/or preferably under the use of a stirrer. Suitable mixing operations are known to the person skilled in the art.

The incubation in step vii. is conducted at conditions suitable for the formation of EDDS. Such conditions are known to the person skilled in the art, preferably those conditions as described herein.

The mixture obtained in step viii. is preferably further processed and the obtained EDDS is preferably purified in step ix., preferably with a method as described herein.

The term "purification" or "purifying" refers to a process in which at least one component, e.g. the obtained EDDS, is separated from at least another component and transferred into a different compartment or phase, wherein the different compartments or phases do not necessarily need to be separated by a physical barrier.

It is preferred in terms of the present invention that the cell disruption step in step iii. includes or is selected from the group consisting of freezing, mechanical disruption and spray-drying or a combination thereof, wherein spray-drying is preferred.

The cell disruption step "freezing" involves the subjection of the obtained cell culture broth or whole-cell biocatalyst to a temperature below 0°C, preferably below -5 °C and especially preferred below -10 °C.

The cell disruption step "mechanical disruption" involves subjecting the obtained cell culture broth or whole-cell biocatalyst to mechanical forces, preferably to high-pressure homogenization at a pressure of at least 500 bar, at least 1000 bar, preferably at least 1500 bar and especially preferably at least 1800 bar.

The cell disruption step "spray-drying" involves the subjection of the obtained cell culture broth or whole-cell biocatalyst to a spray dryer, wherein the inlet temperature of the spray-dryer is preferably at least 60 °C, moreover preferably at least 80 °C, preferably at least 80 °C, moreover preferably at least 100 °C and especially preferably at least 120°C. The spray-drying may also be performed at a temperature of up to 140°C.

Furthermore the cell disruption step in step viii. is preferably selected from a combination of two or three operations selected from the groups consisting of freezing, mechanical disruption and spray-drying.

It is preferred in terms of the present invention that the EDDS synthase expressed by the at least one cell cultivated in step i. comprises or consists of an amino acid sequence selected from the group consisting of SEQ ID NOs.: 1 to 70, or a sequence having at least 87 %, at least 88 %, at least 89 %, at least 90 %, preferably at least 91 %, preferably at least 92 %, preferably at least 93 %, preferably at least 94 %, preferably at least 95 %, preferably at least 96 %, preferably at least 97 %, preferably at least 98 %, preferably at least 99 % to an amino acid sequence selected from the group consisting of SEQ ID NOs.: 1 to 70.

Preferably, the EDDS synthase expressed by the at least one cell cultivated in step i. comprises or consists of a nucleic acid sequence selected from the group consisting of SEQ ID NOs.: 71 to 91, or a sequence having at least 70 %, preferably at least 80 %, preferably at least 85 %, preferably at least 90 %, preferably at least 91 %, preferably at least 92 %, preferably at least 93 %, preferably at least 94 %, preferably at least 95 %, preferably at least 96 %, preferably at least 97 %, preferably at least 98 %, preferably at least 99 % to an amino acid sequence selected from the group consisting of SEQ ID NOs.: 71 to 91.

Whenever the present disclosure relates to the percentage of identity of nucleic acid or amino acid sequences to each other, these values define those values as obtained by using the EMBOSS Water Pairwise Sequence Alignments (nucleotide) program or the EMBOSS Water Pairwise Sequence Alignments (polypeptide) program for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a nucleic acid sequence encoding a polypeptide can be "codon-optimized" if the respective sequence is to be used in another organism in comparison to the original organism a molecule originates from.

It is furthermore preferred in terms of the present invention that the at least one cell expressing an EDDS synthase is selected from the group consisting of *Escherichia coli, Bacillus licheniformis, Bacillus subitilis, Bacillus amyloliquefaciens, Saccharomyces cerevesiae, Komagataella phaffii, Hansenula polymorpha, Yarrowia lipolytica, Kluyveromyces lactis, Corynebacterium glutamicum, Pseudomonas putida, Vibrio natriegens, Aspergillus niger* and *Basfia succiniproducens.*

Further suitable expression organisms are known to the person skilled in the art and include prokaryotic and eukaryotic cells. Preferably, the host cell is a bacterial cell, an archaeal cell, a eukaryotic cell, a fungal cell, a yeast cell, an insect cell or a mammalian cell.

Preferably, the EDDS is expressed in a cell free expression system. Cell-free expression is also called *in vitro* protein expression and is the production of recombinant proteins in solution using the biomolecular translation machinery extracted from cells.

It is preferred that the suitable conditions in step vii. include:
- a temperature of between 20°C and 40°C, preferably of between 30°C and 40°C and/or
- a time of between 4 hours and 7 hours, preferably of between 4.5 hours and 6.5 hours and/or
- a pH in a range of between pH 6 to pH 10, preferably of between pH 7 to pH 9.

Preferably, the pH is adjusted to the desired pH using NaOH or KOH solution.

Furthermore, it is preferred that the biocatalyst mixture obtained in step iv. comprises a maximum of 1 × 10⁷ viable cells, preferably a maximum of 1 × 10⁶, more preferably a maximum of 1 × 10⁵, especially preferably a maximum of 1 × 10³ or especially preferred no viable cells, based on 1 g biocatalyst mixture.

The cell count is preferably determined by standard operation procedures for determining the cell count such as e.g. plate cultivation or flow cytometry. The determined cell count reflects the grade of inactivation of the cells in the biocatalyst mixture.

It is preferred in terms of the present invention that a decreased fumarase activity, preferably no fumarase activity, is detectable during the formation of EDDS in step vii, in comparison to a method without applying a cell disruption in step iii.

The term "decreased fumarase activity" relates to the comparison between a cell or biocatalyst mixture that exhibits fumarase activity before and after applying a cell disruption step. The enzyme fumarase is part of the cell's citrate cycle.

Moreover it is preferred that the purification in step ix. includes or is selected from the group consisting of filtration, crystallization, centrifugation, chromatography, precipitation, or a combination thereof.

Other suitable purification methods are known to the person skilled in the art.

It is preferred in terms of the present invention that the cell disruption step in step iii. is spray-drying and the obtained biocatalyst mixture of step iv. is a powder or granulate having a maximum moisture content of 10 wt.-%, preferably 7 wt.-%, more preferably 3 wt.-% with regard to the total weight of the biocatalyst mixture of step iv.

The remaining moisture content is preferably determined by a method known by the person skilled in the art, e.g. Karl-Fischer titration or loss-on-drying. Preferably the moisture content is determined by loss-on-drying.

Another aspect of the present invention relates to a biocatalyst mixture obtained or obtainable by a method comprising or consisting of steps i. to iv. of a method according to the invention comprising an EDDS synthase and a maximum of 1 × 10⁷ viable cells, preferably a maximum of 1 × 10⁶, more preferably a maximum of 1 × 10⁵, especially preferably a maximum of 1 × 10³ or especially preferred no viable cells, based on 1 g biocatalyst mixture.

It is preferred that the mixture according to the invention comprises a maximum of 1 × 10⁷ viable cells, preferably a maximum of 1 × 10⁶, more preferably a maximum of 1 × 10⁵, especially preferred a maximum of 1 × 10³ or especially preferred no viable cells, selected from the group consisting of *Escherichia coli, Bacillus licheniformis, Bacillus subitilis, Bacillus amyloliquefaciens, Saccharomyces cerevesiae, Komagataella phaffii* and *Hansenula polymorpha, Yarrowia lipolytica, Kluyveromyces lactis, Corynebacterium glutamicum, Pseudomonas putida, Vibrio natriegens, Pseudomonas putida, Aspergillus niger, Basfia succiniproducens* and *Kluyveromyces lactis.*

It is furthermore preferred that the biocatalyst mixture according to the invention is in the form of a spray-dried powder or granulate having a maximum moisture content of 10 wt.-%, preferably 7 wt.-%, more preferably 3 wt.- % with regard to the total weight of the biocatalyst mixture.

Another aspect of the present invention relates to the use of a biocatalyst mixture according to the invention for the manufacturing of EDDS, preferably in a method comprising or consisting of steps v. to ix. of a method according to the invention.

Yet another aspect of the present invention relates to the use of a cell disruption method, preferably selected from freezing, mechanical disruption or spray-drying, for improving the activity of a biocatalyst mixture comprising an EDDS synthase, preferably to obtain a biocatalyst mixture obtained in step iv. of a method according to the invention, and/or for inactivation of the enzyme fumarase expressed by the biocatalyst.

Preferably, the cell-disruption method is spray-drying.

The invention is further characterized in the following by exemplary, non-limiting examples.

### Short description of sequences

SEQ ID NO.: 1 to 70 are amino acid sequences of EDDS synthase variants.
SEQ ID NO.: 71 to 91 are nucleic acid sequences encoding EDDS synthase variants.

### Examples

### Example 1: Generation of a biocatalyst expressing an EDDS synthase

DNA sequences encoding potential EDDS synthases were generated using standard codon usage of *Escherichia coli.* The DNA sequences according to SEQ ID NOs.: 22 and 25 were synthesized (BioCat GmbH) and cloned into the plasmid pDHE19.2 (Ress-Loeschke, M. et al., DE 19848129, 1998, (BASF AG)). The gene of interest lies under control of a rhamnose inducible promoter (rhaBAD). The resulting plasmids were used to transform competent cells (Chung, C.T. et al., Proc Natl Acad Sci USA, 1989, 86, 2172) of the *E. coli* strain TG10, pAgro, pHSG575 (*E. coli* TG10 (Kesseler, M. et al., WO2004050877A1, 2004, (BASF AG)). The strain is a rhaA- derivate of *E. coli* TG1 (DSMZ 6056) transformed with pHSG575 (Takeshita, S. et al., Gene, 1987, 61, 63) and pAgro4 (pBB541 in Tomoyasu, T. et al., Mol. Microbiol., 2001, 40, 397).

### Example 2: Fermentation of biocatalyst

*E. coli* strains producing an EDDS synthase having an amino acid sequence according to SEQ ID NO.:1 or SEQ ID NO.: 2 as obtained in example 1, were cultivated in a small-scale stirred fermentation system. To generate material for inoculation, a seed train consisting of two steps using shake flasks was performed. In a first pre-culture, a 250 mL shake flask filled with 25 mL LB medium was cultivated for 8 h, whereas in the second pre-culture a 1 L shake flask with 100 mL defined mineral salt medium was used. After 16 h, cultivation was stopped and a small portion of the second pre-culture was used to inoculate the main culture.

The main culture fermentation process was a fed-batch process using a defined mineral salt medium. A carbon-vitamin-salt mixture was added using a defined feed profile during cultivation. The pH was controlled via addition of NH₄(OH) and EDDS synthase production was induced by adding a rhamnose-IPTG (0,5 g/L rhamnose and 0.1 mM IPTG) solution at an oxygen transfer rate of 50 mM/h. Dissolved oxygen was actively controlled via stirrer speed and aeration to avoid oxygen limited conditions. After 48 h fermentation was stopped and the whole cell biocatalyst was harvested.

### Example 3: Processing of the biocatalyst

For concentration and for improving the storage stability of the whole cell biocatalyst, the material obtained after fermentation was further processed. During the biocatalyst processing, cell disruption of the whole cell biocatalyst was applied.

The used processing steps were
- freezing
- application of mechanical forces, and/or
- spray drying.

Cell disruption led to a higher EDDS yield compared to the unprocessed enzyme.

### Example 4: Synthesis of EDDS

After cell harvest and downstream processing, the biocatalyst was used for EDDS synthesis. To check EDDS synthase activity, biocatalyst material comprising the EDDS synthase according to SEQ ID NO.: 1 was applied.

### a) Whole cell biocatalyst, no cell disruption

Fumaric acid (9.54 g), magnesium hydroxide (3.56 g) and ethylenediamine (2.50 g) were added to water (60.6 mL) with vigorous stirring and heated to 50°C for 10 min and then allowed to cool to 40°C. The pH of the resulting solution was adjusted to 8.5 with NaOH (25% w/w in water) and then the biocatalyst after fermentation (4.96 g) was added (400 mg cell dry weight, prepared as described above without applying a cell disruption step). The reaction mixture was stirred for 4 h at 40°C. During this time, the pH was adjusted to 8.5 using NaOH (25% w/w in water). After 4 h HPLC analysis showed a concentration of **418 mM EDDS, 46 mM malic acid** and **11 mM fumaric acid.** The substrate and product concentrations over the reaction time are listed below in table 1.

**Table 1: Amounts of substrate (fumaric acid), by-product (malic acid) and product (EDDS) over time in the synthesis of EDDS with whole cell biocatalyst without applying a cell disruption step**

| Time [min] | Fumaric acid [mM] | Malic acid [mM] | EDDS [mM] |
|---|---|---|---|
| 0 | 942 | 0 | 2 |
| 15 | 720 | 6 | 41 |
| 30 | 571 | 16 | 115 |
| 45 | 417 | 27 | 211 |
| 60 | 272 | 35 | 281 |
| 120 | 27 | 52 | 433 |
| 180 | 9 | 52 | 448 |
| 240 | 11 | 46 | 418 |

- Turnover after 240 min: 89%
- Reaction velocity: 4.51 mM/min
- Specific activity: 0.93 kU/g

### b) Biocalayst mixture, mechanical cell disruption

Fumaric acid (9.54 g), magnesium hydroxide (3.56 g) and ethylenediamine (2.50 g) were added to water (63.5 mL) with vigorous stirring and heated to 50°C for 10 min and then allowed to cool to 40°C. The pH of the resulting solution was adjusted to 8.5 with NaOH (25% w/w in water) and then 4.96 g of the biocatalyst mixture, which was subjected to mechanical forces was added (400 mg cell dry weight, prepared as described above). The biocatalyst mixture was treated via high-pressure homogenization at 1800 - 2000 bar (PandaPLUS 2000, GEA, Germany). The reaction mixture was stirred for 5 h at 40°C. During this time the pH was adjusted to 8.5 using NaOH (25% w/w in water). After 5 h HPLC analysis showed a concentration of **456 mM EDDS, 46 mM Malic acid** and **19 mM Fumaric acid.**

**Table 2: Amounts of substrate (fumaric acid), by-product (malic acid) and product (EDDS) over time in the synthesis of EDDS with a biocatalyst mixture processed by mechanical cell disruption**

| Time [min] | Fumaric acid [mM] | Malic acid [mM] | EDDS [mM] |
|---|---|---|---|
| 0 | 933 | 0 | 0 |
| 15 | 436 | 47 | 149 |
| 30 | 231 | 57 | 294 |
| 45 | 100 | 61 | 356 |
| 60 | 53 | 61 | 391 |
| 120 | 18 | 59 | 433 |
| 180 | 19 | 49 | 420 |
| 240 | 19 | 46 | 456 |

- Turnover after 240 min: 97%
- Reaction velocity: 9.59 mM/min
- Specific activity: 1.74 kU/g

### c) Biocatalyst mixture, freezing

Fumaric acid (9.54 g), magnesium hydroxide (3.56 g) and ethylenediamine (2.50 g) were added to water (63.5 mL) with vigorous stirring and heated to 50°C for 10 min and then allowed to cool to 40°C. The pH of the resulting solution was adjusted to 8.5 with NaOH (25% w/w in water) and then 629.33 mg of the biocatalyst, which was subjected to freezing was added (200 mg cell dry weight, prepared as described above). The whole cell biocatalyst was frozen for at least 24 h at -20°C. For EDDS synthesis thawed material was applied.The reaction mixture was stirred for 5 h at 40°C. During this time the pH was adjusted to 8.5 using NaOH (25% w/w in water). After 5 h HPLC analysis showed a concentration of **378 mM EDDS, 156 mM Malic acid** and **9 mM Fumaric acid.**

**Table 3: Amounts of substrate (fumaric acid), by-product (malic acid) and product (EDDS) over time in the synthesis of EDDS with a biocatalyst mixture processed by freezing**

| Time [min] | Fumaric acid [mM] | Malic acid [mM] | EDDS [mM] |
|---|---|---|---|
| 0 | 1117 | 0 | 0 |
| 15 | 791 | 56 | 54 |
| 30 | 610 | 95 | 126 |
| 45 | 441 | 125 | 200 |
| 60 | 286 | 150 | 266 |
| 120 | 42 | 172 | 364 |
| 180 | 15 | 169 | 378 |
| 240 | 12 | 162 | 377 |
| 300 | 9 | 156 | 378 |

- Turnover after 300 min: 77%
- Reaction velocity: 4.38 mM/min
- Specific activity: 1.82 kU/g

### d) Biocatalyst mixture, spray-drying

Fumaric acid (9.54 g), magnesium hydroxide (3.56 g) and ethylenediamine (2.50 g) were added to water (63.5 mL) with vigorous stirring and heated to 50°C for 10 min and then allowed to cool to 40°C. The pH of the resulting solution was adjusted to 8.5 with NaOH (25% w/w in water) and then 209.6 mg of the biocatalyst, which was spray-dried, was added (200 mg cell dry weight, prepared as described above). The whole cell biocatalyst in culture medium was spray-dried with a 2-fluid-nozzle spray dryer (Büchi) at an inlet temperature of 80°C and an outlet temperature of 42 - 44 °C.

The spray-drying conditions were the following:

| System | 2-fluid-nozzle lab scale spray dryer (BÜCHI) |
|---|---|
| Dryer | large |
| Cyclone | large |
| Collecting vessel | 250 mL glass vessel |
| Drying gas (N2) | 30 m³/h |
| Atomization gas (N2) | 60 mm |
| Peristaltic pump | 12 - 15 % |
| Aspirator | 50 % |
| Inlet temperature | 80°C |
| Outlet temperature | 42 - 44°C |
| Feed | Fermentation broth |
| Feed_mass | 250 - 900 g |
| Solids content | 5 - 9% |
| Duration | 120 - 240 min |
| Product yield | 70 - 90% |
| Residual moisture | 3 - 6% |

Dried biocatalyst was used for EDDS synthesis as described above. The reaction mixture was stirred for 5 h at 40°C. During this time the pH was adjusted to 8.5 using NaOH (25% w/w in water). After 5 h HPLC analysis showed a concentration of **432 mM EDDS, 49 mM Malic acid** and **37 mM Fumaric acid.**

**Table 4: Amounts of substrate (fumaric acid), by-product (malic acid) and product (EDDS) over time in the synthesis of EDDS with a biocatalyst mixture processed by spray-drying**

| Time [min] | Fumaric acid [mM] | Malic acid [mM] | EDDS [mM] |
|---|---|---|---|
| 0 | 1155 | 0 | 0 |
| 15 | 928 | 9 | 40 |
| 30 | 845 | 16 | 101 |
| 45 | 678 | 19 | 141 |
| 60 | 584 | 24 | 231 |
| 120 | 276 | 32 | 337 |
| 180 | 133 | 40 | 393 |
| 240 | 59 | 46 | 435 |
| 300 | 37 | 49 | 432 |

- Turnover after 300 min: 87%
- Reaction velocity: 3.53 mM/min
- Specific activity: 1.43 kU/g

### e) Biocatalyst mixture, mechanical disruption and spray-drying

Fumaric acid (9.54 g), magnesium hydroxide (3.56 g) and ethylenediamine (2.50 g) were added to water (63.5 mL) with vigorous stirring and heated to 50°C for 10 min and then allowed to cool to 40°C. The pH of the resulting solution was adjusted to 8.5 with NaOH (25% w/w in water) and then 209.5 mg of the biocatalyst, which was subjected to mechanical cell disruption and spray-drying was added (200 mg cell dry weight, prepared as described above). Harvested fermentation broth containing native whole cell biocatalyst was treated via high pressure homogenization. After homogenization cell broth was dried via spraying. After cell disruption, the cell suspension was used for EDDS synthesis as described above. The reaction mixture was stirred for 5 h at 40°C. During this time the pH was adjusted to 8.5 using NaOH (25% w/w in water). After 5 h HPLC analysis showed a concentration of 458 mM EDDS, 50 mM Malic acid and Fumaric acid 52 mM.

**Table 5: Amounts of substrate (fumaric acid), by-product (malic acid) and product (EDDS) over time in the synthesis of EDDS with a biocatalyst mixture processed by mechanical disruption and spray-drying**

| Time [min] | Fumaric acid [mM] | Malic acid [mM] | EDDS [mM] |
|---|---|---|---|
| 0 | 1150 | 0 | 0 |
| 15 | 920 | 8 | 37 |
| 30 | 804 | 10 | 88 |
| 45 | 651 | 13 | 159 |
| 60 | 561 | 18 | 205 |
| 120 | 240 | 30 | 354 |
| 180 | 124 | 39 | 443 |
| 240 | 67 | 48 | 449 |
| 300 | 52 | 50 | 458 |

- Turnover after 300 min: 93%
- Reaction velocity: 3.35 mM/min
- Specific activity: 1.35 kU/g

### Example 5: Inactivation of cells

After biocatalyst processing, cell viability was analyzed via viable cell determination (= CFU; colony forming units). Samples from various processing steps were used, partially diluted and finally spread on solid growth medium plates. After incubation for 24 h at 37°C bacterial colonies were counted.

**Table 6: Cell viability and cell count (CFU) after different biocatalyst processing methods.**

| | Sample Material | | | |
|---|---|---|---|---|
| | Native | Freezing | Mechanical forces | Spray drying |
| Cell viability | Active | Active | Active | Inactive |
| CFU [1/mL] | >1.0E+08 | >1.0E+07 | >1.0E+05 | 0 |

## Claims

1. Method for producing ethylenediamine-N,N'-disuccinic acid (EDDS) comprising or consisting of the steps:
i. cultivating at least one cell expressing an EDDS synthase,
ii. obtaining a cell culture broth and optionally separating the cell from the culture broth to obtain a whole-cell biocatalyst,
iii. subjecting the obtained cell culture broth or the obtained whole-cell biocatalyst to a cell disruption step,
iv. obtaining a biocatalyst mixture,
v. providing at least one substrate selected from the group consisting of fumaric acid, fumarate, maleic acid, malic acid and maleic anhydrate, or a mixture thereof and ethylenediamine,
vi. mixing the obtained biocatalyst mixture of step iv. with the at least one substrate provided in step v.,
vii. incubating the mixture of step vi. at conditions suitable for the formation of EDDS,
viii. obtaining a mixture containing EDDS,
ix. optionally purifying the obtained mixture of step viii.

2. Method according to claim 1, wherein the cell disruption step in step iii. includes or is selected from the group consisting of freezing, mechanical disruption and spray-drying or a combination thereof, wherein spray-drying is preferred.

3. Method according to claim 1 or 2, wherein the EDDS synthase expressed by the at least one cell cultivated in step i. comprises or consists of an amino acid sequence selected from the group consisting of SEQ ID NOs.: 1 to 70, or a sequence having at least 87 %, at least 88 %, at least 89 %, at least 90 %, preferably at least 91 %, preferably at least 92 %, preferably at least 93 %, preferably at least 94 %, preferably at least 95 %, preferably at least 96 %, preferably at least 97 %, preferably at least 98 %, preferably at least 99 % to an amino acid sequence selected from the group consisting of SEQ ID NOs.: 1 to 70.

4. Method according to any one of the preceding claims, wherein the at least one cell expressing an EDDS synthase is selected from the group consisting of *Escherichia coli, Bacillus licheniformis, Bacillus subitilis, Bacillus amyloliquefaciens, Saccharomyces cerevesiae, Komagataella phaffii, Hansenula polymorpha, Yarrowia lipolytica, Kluyveromyces lactis, Corynebacterium glutamicum, Pseudomonas putida, Vibrio natriegens, Aspergillus niger* and *Basfia succiniproducens.*

5. Method according to any one of the preceding claims, wherein the suitable conditions in step vii. include:
- a temperature of between 20°C and 40°C, preferably of between 30°C and 40°C and/or
- a time of between 4 hours and 7 hours and/or
- a pH in a range of between pH 6 to pH 10, preferably of between pH 7 to pH 9.

6. Method according to any one of the previous claims, wherein the biocatalyst mixture obtained in step iv. comprises a maximum of 1 × 10⁷ viable cells, preferably a maximum of 1 × 10⁶, more preferably a maximum of 1 × 10⁵, especially preferably a maximum of 1 × 10³ or especially preferably no viable cells, based on 1 g biocatalyst mixture.

7. Method according to any one of the previous claims, wherein a decreased fumarase activity, preferably no fumarase activity, is detectable during the formation of EDDS in step vii, in comparison to a method without applying a cell disruption in step iii.

8. Method according to any one of the preceding claims, wherein the purification in step ix. includes or is selected from the group consisting of filtration, crystallization, centrifugation, chromatography, precipitation, or a combination thereof.

9. Method according to any one of the preceding claims, wherein the cell disruption step in step iii. is spray-drying and the obtained biocatalyst mixture of step iv. is a powder or granulate having a maximum moisture content of 10 wt.-%, preferably 7 wt.-%, more preferably 3 wt.-% with regard to the total weight of the biocatalyst mixture of step iv.

10. Biocatalyst mixture obtained or obtainable by a method comprising or consisting of steps i. to iv. of a method according to claims 1 to 9 comprising an EDDS synthase and a maximum of 1 × 10⁷ viable cells, preferably a maximum of 1 × 10⁶, more preferably a maximum of 1 × 10⁵, especially preferably a maximum of 1 × 10³ or especially preferably no viable cells, based on 1 g biocatalyst mixture.

11. Biocatalyst mixture according to claim 10, wherein the biocatalyst mixture comprises a maximum of 1 × 10⁷ viable cells, preferably a maximum of 1 × 10⁶, more preferably a maximum of 1 × 10⁵, especially preferably a maximum of 1 × 10³ or especially preferably no viable cells, selected from the group consisting of *Escherichia coli, Bacillus licheniformis, Bacillus subitilis, Bacillus amyloliquefaciens, Saccharomyces cerevesiae, Komagataella phaffii and Hansenula polymorpha, Yarrowia lipolytica, Kluyveromyces lactis, Corynebacterium glutamicum, Pseudomonas putida, Vibrio natriegens, Pseudomonas putida, Aspergillus niger, Basfia succiniproducens and Kluyveromyces lactis.*

12. Biocatalyst mixture according to claim 10 or 11, wherein the biocatalyst mixture is in the form of a spray-dried powder or granulate having a maximum moisture content of 10 wt.-%, preferably 7 wt.-%, more preferably 3 wt.- % with regard to the total weight of the biocatalyst mixture.

13. Use of a biocatalyst mixture according to claim 10 to 12 for the manufacturing of EDDS, preferably in a method comprising or consisting of steps v. to ix. of a method according to claim 1 to 9.

14. Use of a cell disruption method, preferably selected from freezing, mechanical disruption or spray-drying, for improving the activity of a biocatalyst mixture comprising an EDDS synthase, preferably to obtain a biocatalyst mixture obtained in step iv. of a method according to claims 1 to 9, and/or for inactivation of the enzyme fumarase expressed by the biocatalyst.

15. Use according to claim 13 or 14, wherein the cell-disruption method is spray-drying.
